Europäisches Patentamt

(19) European Patent Office

Office européen des brevets

(11) **EP 0 569 268 B1**

(12) **FASCICULE DE BREVET EUROPEEN**

(45) Date de publication et mention
de la délivrance du brevet:
**09.07.1997 Bulletin 1997/28**

(51) Int. Cl.$^6$: **B01J 29/70**, B01J 29/74

(21) Numéro de dépôt: **93401081.0**

(22) Date de dépôt: **26.04.1993**

(54) **Catalyseur à base de zéolithe oméga renfermant au moins un métal des groupes IIA,IVB,IIB ou IVA et son utilisation en isomérisation d'une coupe C8 aromatique**

Katalysator auf Basis eines Omega-Zeolithe und mindestens ein Metall der Gruppe IIA enthaltend IVB, IIB oder IVA und seine Anwendung für die Isomerisation einer aromatischen C8-Fraktion

Omega zeolite catalyst containing at least one metal of the group IIA, IVB, IIB or IVA and the use thereof in the isomerisation of a C8 fraction

(84) Etats contractants désignés:
**DE FR GB IT NL**

(30) Priorité: **06.05.1992 FR 9205680**

(43) Date de publication de la demande:
**10.11.1993 Bulletin 1993/45**

(73) Titulaire: **INSTITUT FRANCAIS DU PETROLE**
**92502 Rueil-Malmaison (FR)**

(72) Inventeurs:
 • **Benazzi, Eric**
  **F-78360 Montesson (FR)**
 • **Travers, Christine**
  **F-92500 Rueil Malmaison (FR)**

 • **Choplin, Agnès**
  **F-69100 Villeurbanne (FR)**
 • **Joly, Jean-Francois**
  **F-75003 Paris (FR)**
 • **Basset, Jean-Marie**
  **F-69100 Villeurbanne (FR)**

(74) Mandataire: **Andreeff, François**
 **INSTITUT FRANCAIS DU PETROLE**
 **4, avenue de Bois-Préau**
 **92502 Rueil-Malmaison (FR)**

(56) Documents cités:
 EP-A- 0 007 126          EP-A- 0 206 871
 WO-A-90/09845          GB-A- 2 096 481

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un catalyseur de type alumino-silicate comprenant une zéolithe oméga (de type structural MAZZITE) dont on a modifié la sélectivité géométrique et/ou les propriétés catalytiques par dépôt sur la surface extérieure de ses cristaux d'au moins un métal choisi parmi les métaux des groupes IIa (Be, Mg, Ca, Sr, Ba, Ra), IVb (Ti, Zr, Hf), IIb (Zn, Cd, Hg) et IVa (Ge, Sn, Pb) de la classification périodique des éléments, eventuellement au moins un métal du groupe VIII de ladite classification et une matrice et son utilisation dans les réactions d'isomérisation des hydrocarbures $C_8$ aromatiques. Elle concerne également le procédé de préparation de ladite zéolithe oméga modifiée.

Actuellement, les catalyseurs utilisés industriellement dans ces réactions sont essentiellement à base de zéolithe ZSM-5, seule ou en mélange avec d'autres zéolithes, telles que la mordénite par exemple. Ces catalyseurs sont notamment décrits dans les brevets US-A-4467129, et US-A-4482773. L'intérêt de la zéolithe ZSM5 réside dans son excellente sélectivité de forme, qui conduit à une grande sélectivité en paraxylène, la sélectivité vis-à-vis des réactions secondaires indésirables de dismutation restant à un niveau très faible.

D'autres zéolithes à ouverture de pores plus large 12MR (ouverture à 12 atomes d'oxygène) ont aussi été utilisées, telle que la mordénite et la zéolithe oméga. Les catalyseurs à base de mordénite ou de zéolithe oméga sont notamment décrits dans les brevets US 4723051 et US 4665258. Cependant, ces zéolithes ne possèdent pas de propriétés de sélectivité géométrique particulières. Ceci se traduit, quelque soit son rapport Si/Al, par des sélectivités en paraxylène plus faibles que celles obtenues pour la zéolithe MFI et surtout par des productions de triméthylbenzènes très importantes. La demande de brevet WO90/09845 apporte une solution qui permet de diminuer le taux de dismutation des xylènes dans les cas de catalyseurs à base de mordénite, par greffage de composés organométalliques sur la mordénite.

La zéolithe oméga est une zéolithe très active pour la réaction d'isomérisation des $C_8$ aromatiques. Néanmoins, la production de triméthylbenzènes par dismutation est en effet plus importante dans la zéolithe oméga que dans la ZSM5.

L'invention concerne un catalyseur renfermant une matrice, au moins un métal du GVIII et une zéolithe oméga, dans lequel ladite zéolithe oméga renferme au moins un métal choisi parmi les métaux du groupe IIa, IVb, IIb et IVa de la classification périodique des élèments et est telle que son rapport atomique Si/Al global soit compris entre 2.5 et 100 et sa teneur pondérale en sodium par rapport au poids de zéolithe oméga sèche est inférieure à 2000 ppm, ledit métal (ou lesdits métaux) des groupes IIa, IIb, IVa, IVb étant déposé sur la zéolite oméga par greffage avec au moins un composé organométallique dudit métal (desdits métaux).

La demanderesse a en effet découvert qu'il est possible, grâce au dépôt sur la surface extérieure des cristaux de la zéolithe oméga d'au moins un métal choisi parmi les métaux du groupe IIa, IVb, IIb et IVa et en particulier de magnésium, de titane, de zinc ou/et d'étain, d'obtenir des catalyseurs actifs et sélectifs pour la réaction d'isomérisation des $C_8$ aromatiques. Cette procédure de préparation nouvelle confère à la zéolithe oméga des propriétés de sélectivité géométrique très améliorées, ce qui se traduit par une inhibition importante des réactions secondaires indésirables telles que la dismutation. Cette nouvelle zéolithe oméga modifiée conduit par ailleurs à des sélectivités vis-à-vis des réactions parasites de désalkylation inférieures à celles des catalyseurs basés sur la ZSM5. On aboutit ainsi à des solides qui présentent des performances en isomérisation des $C_8$ aromatiques non seulement meilleures que celles des zéolithes oméga de l'art antérieur, mais également au moins équivalentes, voire supérieures, aux performances des catalyseurs à base de ZSM5.

D'autre part, il a été découvert de façon surprenante que la régénération du catalyseur à base de zéolithe oméga, ayant subi sur la surface externe des cristaux le dépôt d'au moins un métal choisi parmi les métaux du groupe IIa, IVb, IIb et IVa, permet d'obtenir une activité plus grande dans ce cas ci.

Avant de sélectiver le réseau poreux de la zéolithe oméga par dépôt d'au moins un métal des groupes IIa, IVb, IIb, IVa, il est préférable d'utiliser la forme $H^+$ ou $NH_4^+$ de ladite zéolithe oméga avec une teneur pondérale en sodium par rapport au poids de zéolithe oméga sèche généralement inférieure à 2000 ppm, de préférence à 1000 ppm et, de manière encore plus préférée, à 500 ppm, un rapport Si/Al atomique compris entre 2.5 et 100, et de préférence entre 3 et 25, une capacité d'absorption d'azote mesurée à -196°C sous une pression partielle $P/P_o=0.19$, supérieure à 0.096 $cm^3$ liquide/gramme. La teneur pondérale en eau de la zéolithe oméga forme $H^+$ ou $NH4^+$ est habituellement comprise entre entre 5 et 50%.

Pour préparer ladite forme $H^+$ ou $NH4^+$ on pourra employer toute technique connue de l'homme du métier, comme, par exemple, l'attaque acide directe, la calcination en présence ou non de vapeur d'eau de la forme $NH4^+$ suivie d'une ou plusieurs attaques acides, les cycles "calcination(s) - attaque(s) acide(s)".

Le dépôt d'au moins un des métaux choisis dans l'ensemble formé par les métaux des groupes IIa, IVb, IIb et IVa est réalisé avantageusement par greffage en utilisant comme agent de greffage au moins un composé organométallique dudit métal qui est, d'une part, suffisamment volumineux pour ne pas pénétrer à l'intérieur du réseau microporeux de la zéolithe oméga et, d'autre part , susceptible de réagir avec les groupes OH de surface. On pourra notamment employer comme agents de greffage, dans le cas de l'étain, les composés de formule $SnR^1R^2R^3R^4$ dans lesquels les

groupes $R^1$, $R^2$, $R^3$ et $R^4$ identiques ou différents, sont des groupes organiques d'encombrement varié et, en général, d'encombrement important ; à titre d'exemples non limitatifs, on peut citer les radicaux alkyl, aryl, organosilyl vinyl, polynucléaire aryl, cyclo-alkyl, allyl, propargyl. Les groupes $R^i$, i=1,2,3 ou 4, peuvent également être un groupe de type alkoxy ou aryl-oxy encombré, comme par exemple un groupe ter-butoxy, o,o'-diphényl-phénoxy, o,o'-di-iopropyl phénoxy etc...

Le groupement $R^i$ peut aussi être un hydrure, mais il reste au moins un groupement $R^i$ organique fixé sur l'étain, à titre d'exemples non limitatifs on peut citer dans le cas de l'étain les composés $SnBu_2H_2$, $SnBu_3H$.

Dans le cas général des autres métaux du groupe IIa (Be, Mg, Ca, Sr, Ba, Ra) et des métaux des groupes IVb (Ti, Zr, Hf), IIb (Zn, Cd, Hg) et IVa (Ge, Sn, Pb), on pourra utiliser, comme agents de greffage, également des composés de formule $BeR_2$, $MgR_2$, $CaR_2$, $SrR_2$, $BaR_2$, $RaR_2$, $TiR_4$, $ZrR_4$, $HfR_4$, $CdR_2$, $HgR_2$, $GeR_4$, $SnR_4$ ou $PbR_4$ dans lesquels $R^i$, identiques ou différents entre eux, sont des groupes organiques tels que ceux définis précédemment.

A titre d'exemples non limitatifs, dans le cas de l'étain un agent de greffage préféré est le tétrabutylétain noté $SnBu_4$. Dans le cas du magnésium un agent de greffage préféré est le bis-néopentylmagnésium noté $MgNp_2$, dans le cas du zirconium un agent de greffage préféré est le tétranéopentylzirconium noté $ZrNp_4$, et dans le cas du germanium, un agent de greffage préféré est le tetrabutylgermanium noté $GeBu_4$.

Les étapes de préparation de la zéolithe greffée sont les suivantes :

Prétraitement de la zéolithe oméga

La zéolithe est prétraitée par l'une des deux méthodes décrites ci-aprés :

- Activation sous flux de gaz inerte ($N_2$ par exemple).

Généralement après une mise sous gaz inerte à une température comprise entre environ 50°C et 200°C, de préférence égale à environ 150°C, pendant 8 à 24 heures, par exemple pendant environ 12 heures (étape de séchage), on procède à une calcination de la zéolithe oméga à une température comprise entre 410°C et 580°C, de préférence égale à environ 550°C pour une durée comprise entre 1 et 8 heures, de préférence égale à environ 4 heures, sous mélange gazeux de gaz inerte et d'air. Une diminution de la température, sous gaz inerte jusqu'à une température comprise entre 5°C et 35°C, de préférence égale à environ 20°C, est ensuite réalisée.

- Prétraitement sous vide

La zéolithe oméga est chauffée à une température comprise entre 120°C et 200°C, de préférence égale à 160°C durant un temps suffisamment long pour obtenir un vide de $133.10^{-4}$ Pa. La température est alors augmentée jusqu'à une valeur comprise entre 400 et 500°C, de préférence égale à environ 450°C. La zéolithe oméga ainsi activée est refroidie sous vide à une température comprise entre 5°C et 35°C et de préférence égale à environ 20°C, puis conservée sous atmosphère inerte.

Greffage de la zéolithe oméga

Au moins un agent de greffage peut être fixé sur la zéolithe oméga par une voie en phase gazeuse ou bien par une voie en phase liquide. Dans ce dernier cas, le composé organométallique choisi est alors placé en solution dans un solvant, par exemple l'hexane, sous gaz inerte.

A titre d'exemple illustratif et non limitatif dans le cas du $SnBu_4$, on utilise l'une des procédures suivantes, sachant que l'on emploiera des méthodes analogues dans le cas des autres agents de greffage cités précédemment. les suivantes :

- Greffage par voie en phase gazeuse

La zéolithe prétraitée selon les modes décrits précédemment est mise sous vide statique ( $133.10^{-4}$ Pa) à 25°C, dans un récipient clos. Le composé organométallique, dans ce cas $SnBu_4$, est injecté à 25°C par l'intermédiaire d'un septum.

La température à laquelle s'effectue le greffage est avantageusement supérieure à environ 50°C et, de préférence comprise entre 100°C et 400°C et, par exemple égale à environ 150°C pour le $SnBu_4$. Pour d'autres compo-sés et à titre d'exemple non limitatif, on utilise une température de égale à environ 250°C pour le $GeBu_4$, égale à environ 150°C pour le $MgNp_2$ et égale à environ 80°C pour le $ZrNp_4$.

Les durées de traitement sont habituellement supérieures à 1 minute et sont avantageusement comprises entre 1 heure et 24 heures et de préférence égale à environ 12 heures. Après greffage, l'excès de $Sn(Bu)_4$ présent dans le système est éliminé généralement par une purge sous vide dynamique ou sous gaz inerte.

- Greffage par voie en phase liquide

La zéolithe oméga est mise en suspension dans un solvant de l'agent de greffage constitué par au moins un alcane ayant au moins 5 atomes de carbone, par exemple l'hexane, sous gaz inerte. Le volume V d'hexane engagé (en ml) est compris entre 1 fois et 50 fois le poids de zéolithe oméga séche engagée, soit un V/P compris entre 1 et 50, de préférence égal à environ 3. Puis l'agent de greffage, par exemple, le $SnBu_4$ est introduit. La température à laquelle s'effectue la mise en contact de la zéolithe et de l'agent de greffage est avantageusement supérieure à la température de congélation du solvant utilisé (-95°C dans le cas de l'hexane) et, de préférence comprise entre environ 5°C et 35°C. Les durées de traitement sont habituellement supérieures à 1 minute et sont avantageusement comprises entre environ 5 et 60 minutes et de préférence égal à environ 15 minutes. Ce mode opératoire est applicable aux autres organométalliques précédemment cités. Le solvant est alors éliminé sous vide ou sous gaz inerte puis la température est augmentée afin de réaliser le greffage. La température à laquelle s'effectue le greffage est de préférence comprise entre 100°C et 400°C et par exemple égale à environ 150°C dans le cas du $SnBu_4$. Pour d'autres composés et à titre d'exemple non limitatif, on utilise une température égale à environ 250°C pour le $GeBu_4$, égale à environ 150°C pour le $MgNp_2$ et égale à environ 80°C pour le $ZrNp_4$. Les durées de traitement sont comprises entre 1 heure et 24 heures et de préférence égale à environ 12 heures.

Après greffage, l'excès de $SnBu_4$ est éliminé par un ou plusieurs rinçages avec un solvant tel que celui employé précédemment.

Décomposition des fragments organiques

Le traitement thermique de décomposition des fragments organiques se fait par l'une des méthodes suivantes :

- Décomposition des fragments organiques sous atmosphère oxydante

  Ce traitement est avantageusement réalisé en présence d'oxygène (de préférence en mélange avec un gaz inerte tel que l'azote) à une température supérieure à 150°C, de préférence comprise entre 350°C et 550°C et environ égale à 450°C. Ce mode opératoire de décomposition est valable pour tous les autres agents de greffage cités précédemment.

- Décomposition des fragments organiques sous vide

  Ce traitement est avantageusement réalisé à une température comprise entre 350°C et 550°C, de préférence égale à environ 450°C, sous vide dynamique durant quatre heures. Puis, la zéolithe oméga greffée est maintenue à cette température sous flux de gaz inerte pendant une durée comprise entre 10 et 20 heures et de préférence égale à environ 15 heures. Ce mode opératoire convient pour tous les autres agents organométalliques décrits précédemment.

Le dépôt éventuel d'au moins un autre métal choisi parmi les métaux des groupes IIa, IVb, IIb ou IVa à la place ou en plus du métal greffé selon les modes opératoires présentés précédemment s'effectue de manière analogue.

A l'issue du traitement thermique de décomposition, la teneur pondérale de la zéolithe oméga en métaux choisis parmi l'un des groupes IIa, IVb, IIb et/ou IVa est comprise entre 0.01 et 5% et, de manière avantageuse entre 0.01 et 4%.

Néanmoins, on peut éventuellement régler le niveau de sélectivité de la zéolithe oméga en procédant au besoin à un ou plusieurs cycles supplémentaires "greffage de métal (métaux) des groupes IIa, IVa, IIb ou IVb, calcination", suivant une des techniques décrites précédemment, de manière à atteindre des teneurs pondérales en métal (métaux) desdit groupes (par exemple magnésium) comprises entre 0,01 et 25%, de préférence supérieures à 5% et, si nécessaire, à 7%.

Les propriétés acides moyennes et globales de la zéolithe oméga ne sont pas altérées par le dépôt dudit (desdit) métal (métaux) selon les techniques décrites ci-dessus.

Ainsi, la zéolithe oméga obtenue possède une teneur pondérale en sodium par rapport au poids de zéolithe oméga séche inférieure à 2000 ppm, de préférence à 1000 ppm et, de manière encore plus préférée, à 500 ppm, un rapport atomique Si/Al global compris entre 2.5 et 100 et, de préférence entre 3 et 25.

Les caractéristiques de la zéolithe oméga peuvent être mesurées par les méthodes suivantes:

- les rapports atomiques Si/Al sont déterminés par fluorescence X et par résonance magnétique nucléaire du silicium 29, les teneurs en sodium par absorption atomique.
- le volume de maille élémentaire et la cristallinité sont déterminés par diffraction X, l'échantillon de zéolithe oméga étant préparé comme dans le mode opératoire de la norme ASTM D3942 80 établie pour la faujasite.

La zéolithe oméga peut être soumise (avant ou après le dépôt d'au moins un métal choisi parmi les métaux des groupes IIa, IVb, IIb et IVa) au dépôt d'au moins un métal du groupe VIII de préférence choisi dans le groupe formé par le platine et le palladium, et mise en forme par toute technique connue de l'homme du métier. Elle peut en particulier être mélangée à une matrice, généralement amorphe, par exemple à une poudre humide de gel d'alumine. Le mélange est ensuite mis en forme, par exemple par extrusion au travers d'une filière. La teneur en zéolithe oméga du mélange ainsi obtenu est généralement comprise entre 0,5 et 99,99% et avantageusement comprise entre 40 et 90% en poids par rapport au mélange (zéolithe oméga + matrice). Elle est plus particulièrement comprise entre environ 60 et 85 % en poids par rapport au mélange (zéolithe + matrice). La teneur en matrice du catalyseur est généralement comprise entre 0,01 et 99,5 %, avantageusement entre environ 10 et 60 % et, de préférence, entre environ 15 et 40 % en poids par rapport au mélange (zéolithe oméga + matrice).

Dans la suite du texte on désignera par le terme support le mélange zéolithe oméga (sur laquelle aucun métal n'a été greffé) + matrice.

La mise en forme peut être réalisée avec d'autres matrices que l'alumine, telles que, par exemple, la magnésie, la silice alumine, les argiles naturelles (kaolin, bentonite) et par d'autres techniques que l'extrusion, telles que le pastillage ou la dragéification.

Le métal hydrogénant du groupe VIII, de préférence Pt et/ou Pd, peut également être déposé sur le support par tout procédé connu de l'homme de l'art et permettant le dépôt du métal sur la zéolithe oméga. On peut utiliser la technique d'échange cationique avec compétition, où l'agent compétiteur est de préférence le nitrate d'ammonium, le rapport de compétition étant au moins égal à environ 50 et avantageusement d'environ 50 à 200. Dans le cas du platine ou du palladium, on utilise habituellement un complexe tétramine du platine ou un complexe tétramine du palladium ; ces derniers se déposeront alors pratiquement en totalité sur la zéolithe oméga. Cette technique d'échange cationique peut également être utilisée pour déposer directement le métal sur la poudre de zéolithe oméga, avant son mélange éventuel avec une matrice.

Le dépôt du métal (ou des métaux) du groupe VIII est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures ; de préférence on opérera entre 350 et 550°C pendant 2 à 5 heures. La teneur en métal du groupe VIII (de préférence Pt et/ou Pd) déposé sur le catalyseur est obtenue à l'issue de l'échange est comprise habituellement entre 0,05 et 1,5 %, de préférence entre 0,1 et 1 %, en poids par rapport à l'ensemble du catalyseur.

On peut également déposer le platine et/ou le palladium non plus directement sur la zéolithe oméga, mais sur le liant, avant ou après l'étape de mise en forme, en mettant en oeuvre un échange anionique avec de l'acide hexachloroplatinique, de l'acide hexachloropalladique et/ou du chlorure de palladium en présence d'un agent compétiteur, par exemple l'acide chlorhydrique. En général, après dépôt de platine et/ou de palladium, le catalyseur est comme précédemment soumis à une calcination puis réduit sous hydrogène comme indiqué ci-dessus.

Le catalyseur bifonctionnel obtenu par les procédures précédentes peut être mis en oeuvre notamment dans les réactions d'isomérisation d'une coupe $C_8$ aromatique, comprenant par exemple soit uniquement un mélange de xylènes, soit un mélange de xylène(s) et d'éthylbenzène. L'isomérisation des alkyl-aromatiques, et en particulier des xylènes, revêt une importance commerciale considérable. C'est en général surtout le paraxylène qui est le produit le plus recherché, car il est notamment utilisé comme intermédiaire dans la fabrication des fibres polyesters. On préfère fabriquer le paraxylène en isomérisant le métaxylène, qui lui peut être obtenu par isomérisation de l'orthoxylène. L'éthylbenzène, qui est difficilement séparable par distillation du mélange des xylènes (les points d'ébullition des différents composés sont très proches), se trouve très souvent dans la charge d'isomérisation des hydrocarbures aromatiques en $C_8$.

Les conditions opératoires du procédé d'isomérisation d'une coupe $C_8$ aromatique effectué en présence d'au moins un catalyseur selon l'intervention sont habituellement les suivantes :

- température comprise entre 270 et 600°C, de préférence entre 350 et 510°C,
- pression comprise entre 0,5 et 100 bars, de préférence entre 2 et 30 bars,
- vitesse spatiale (pph), en masse de charge par unité de charge de catalyseur et par heure, comprise entre 0,5 et 200, de préférence entre 2 et 100,
- rapport molaire hydrogène sur hydrocarbures de la charge ($H_2$/HC) compris entre 0,5 et 12, de préférence entre 2 et 6.

Les exemples qui suivent illustrent l'invention sans toutefois en limiter la portée ; ils sont donnés pour une charge formée de 80 % d'orthoxylène et de 20 % d'éthylbenzène (% en poids).

Exemple 1 : Catalyseurs A1 et A2, greffés à l'étain, et conformes à l'invention.

La matière première utilisée est une zéolithe oméga, qui possède un rapport atomique Si/Al global égal à 3.2, une teneur pondérale en sodium par rapport au poids en zéolithe oméga sèche d'environ 5.3 %, un volume de maille élémentaire de 2,196 $nm^3$, et un volume poreux, à l'azote, mesuré à -196°C et à $P/P_o$=0.19 de 0.125 $cm^3$ liquide par gramme.

Cette zéolithe oméga subit tout d'abord une calcination dite sèche à 550°C sous flux d'air et d'azote durant 6 heures. Puis, le solide obtenu est soumis à trois échanges ioniques dans une solution de $NH_4NO_3$ 10N à environ 100°C pendant 4 heures, pour chaque échange. La zéolithe oméga est alors soumise à un traitement hydrothermique, en présence de 50% de vapeur d'eau à 625°C, durant 4 heures. La zéolithe subit alors une attaque acide, à l'aide d'acide nitrique 1.5N, à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10). La zéolithe oméga est ainsi désaluminée selon le mode opératoire décrit dans le brevet EP 214042.

A l'issue de ces traitements, la zéolithe oméga sous forme H a un rapport Si/Al atomique global égal à 11.3, un rapport Si/Al global de charpente, déterminé par RMN du $^{29}$Si de 12, une teneur pondérale en sodium par rapport au poids de zéolithe oméga sèche de 160 ppm, un volume de maille élémentaire de 2,145 nm$^3$, et une capacité d'adsorption en azote mesurée à -196°C et à $P/P_o$=0.19 de 0.124 cm$^3$ liquide/g.

L'étain est ensuite greffé sur la surface externe des cristaux de la zéolithe oméga forme H par la voie en phase liquide décrite précédemment. La décomposition des fragments organiques liés à l'étain après l'étape de greffage peut se faire sous vide ou bien en présence d'air (atmosphère oxydante).

### i) Méthode de greffage en phase liquide et décomposition des fragments organiques sous vide.

On opère selon les étapes successives suivantes :

- Activation de la zéolithe oméga, par traitement sous gaz inerte à 150°C durant 12 heures, puis diminution de la température à 20°C, sous inerte.
- Calcination des éventuelles traces de composés organiques adsorbés dans la zéolithe sous flux d'air sec (0.4 l/h/g) et de gaz inerte (2 l/h/g) durant 2 heures à 550°C, puis uniquement sous flux d'air sec (2 l/h/g) durant 2 heures toujours à 550°C.
- La zéolithe oméga est refroidie à 20°C sous gaz inerte et mise en suspension dans l'hexane. Le tétrabutylétain est alors injecté, toujours sous atmosphère de gaz inerte.
- Après 15 minutes d'agitation, le solvant est éliminé sous vide. La température de la zéolithe oméga est alors augmentée jusqu'à 150°C, puis maintenue durant 6 heures, sous atmosphère inerte. Après refroidissement à température ambiante de la zéolithe, l'excès de SnBu$_4$ est éliminé par rinçage avec une solution d'hexane frais.
- Les fragments organiques butyles liés à l'étain sont alors décomposés par traitement thermique sous vide. Pour cela, la zéolithe oméga est placée sous vide dynamique durant 2 heures à la température de 450°C, puis sous flux de gaz inerte durant 15 heures.

Le solide obtenu à l'issue de ces traitements est référencé OM1 : sa teneur pondérale en étain est de 1.3%.

### ii) Méthode de greffage en phase liquide et décomposition des fragments organiques sous atmosphère oxydante

Le mode d'obtention de la zéolithe oméga et son mode de greffage sont identiques à ceux décrits précédemment conduisant à la préparation du solide référencé OM1. Dans cet exemple seul change le mode de décomposition des fragments organiques butyles liés à l'étain qui se fait sous atmosphère oxydante.

Ce traitement est réalisé en présence d'un mélange d'oxygène et de gaz inerte, à 450°C durant 4 heures.

Le solide obtenu à l'issue de ces traitements est référencé OM2 : sa teneur pondérale en étain est de 1.26%.

Les deux solides OM1 et OM2 subissent ensuite les memes traitements : ils sont chacun mélangés intimement avec de l'alumine sur laquelle 0.3% en poids de platine sont dispersés. Le support constitué par le mélange zéolithe oméga OM1-alumine (ou zéolithe oméga OM2-alumine) contient 39% en poids d'alumine. La teneur pondérale en platine de chacun des catalyseurs finaux A1 (contenant OM1) et A2 (contenant OM2) est donc d'environ 0.12 %.

Les catalyseurs ainsi fabriqués sont ensuite mis en forme par pastillage, calcinés sous air à 500°C durant 2 heures et réduits sous hydrogène à 500°C pendant 3 heures.

Ces catalyseurs A1 et A2 sont alors testés en isomérisation du mélange orthoxylène (80% poids) et éthylbenzène (20% poids), à une température de 410°C, sous une pression de 12 bars, avec une vitesse spatiale (pph) de 10 (heure)$^{-1}$ et un rapport molaire hydrogène sur hydrocarbures (H$_2$/HC) de 4 environ.

Les performances des catalyseurs A1 et A2 (et des catalyseurs préparés dans les exemples suivants), reportées dans le tableau I, sont définies par

$$\text{Conversion de l'o-xylène(\%)} = \frac{\text{Masse d'o-xylène dans la charge - masse d'o-xylène dans la recette}}{\text{Masse d'o-xylène dans la charge}} \times 100$$

$$\text{Sélectivité en isomérisation (\%)} = \frac{\text{Masse m-xylène + masse p-xylène}}{\text{Masse de produits}} \times 100$$

$$\text{Approche à l'équilibre de l'ortho-xylène (AEQ-ox) (\%)} = \frac{\text{Nombre de moles d'ortho-xylène dans la recette}}{\text{Nombre de moles d'ortho-xylène à l'équilibre thermodynamique}} \times 100$$

$$\text{Rendement en } C_8 \text{ aromatique (\%)} = \frac{\text{Masse de } C_8 \text{ aromatiques et de naphtènes dans la recette}}{\text{Masse totale de } C_8 \text{ aromatiques dans la charge}} \times 100$$

$$\text{Sélectivité en dismutation (\%)} = \frac{\text{Masse de triméthylbenzène + masse toluène + masse benzène}}{\text{Masse des produits}} \times 100$$

$$\text{Sélectivité en déalkylation (\%)} = \frac{\text{Masse de benzène}}{\text{Masse de produits}} \times 100$$

$$\text{Sélectivité en craquage (\%)} = \frac{\text{Masse des gaz de C1 à C4}}{\text{Masse des produits}} \times 100$$

Le catalyseur A1 après test catalytique est régénéré selon le mode opératoire suivant;

Sous flux d'azote pur (3 l/g/h) et à la pression atmosphérique, la température du catalyseur est augmentée jusqu'à 150°C à raison de 3°C/min. Puis, un mélange gazeux air sec/azote (50/50 en poids) est injecté à la place du flux d'azote. La température est alors augmentée jusqu'à 550°C à raison de 3°C/min. Après un palier de 15 minutes, le mélange gazeux (air/azote) est remplacé uniquement par un flux d'air sec (3 l/h/g). Un palier de deux heures à la température de 550°C est alors effectué. La température est ensuite diminuée jusqu'à 300°C sous flux d'air sec, puis jusqu'à la température ambiante sous azote.

Le catalyseur subit alors une étape de réduction sous hydrogène dans les conditions suivantes: sous une pression d'hydrogène de 12 bars et une pph égale à 5 en hydrogène, le catalyseur est chauffé, à raison de 3°C/min jusqu'à 150°C, température à laquelle un palier de 2 heures est effectué. La meme opération est réalisée à la température de 250°C, 350°C et 450°C, la vitesse de montée en température entre chaque palier est de 3°C/min. Le catalyseur est ensuite refroidi sous hydrogène jusqu'à température ambiante.

Le catalyseur régénéré A1R ainsi obtenu est de nouveau testé en isomérisation du mélange ortho-xylène (80% poids) et éthylbenzène (20% poids), à une température de 410°C, sous une pression de 12 bars, avec une vitesse spatiale (pph) de 50 (heure)$^{-1}$ et un rapport molaire hydrogène sur hydrocarbures ($H_2$/HC) de 4 environ. Les performances du catalyseur A1R sont reportées dans le tableau II.

Exemple 2 : Catalyseur A3 greffé à l'étain, conforme à l'invention.

La matière première utilisée est la même zéolithe oméga que celle utilisée dans l'exemple 1. Elle possède un rapport atomique Si/Al global égal à 3.2, une teneur pondérale en sodium par rapport au poids en zéolithe oméga sèche d'environ 5.3 %, un volume de maille élémentaire de 2,196 nm$^3$, et un volume poreux, à l'azote, mesuré à -196°C et à P/P$_o$=0.19 de 0.125 cm$^3$ liquide par gramme.

Néanmoins, dans cet exemple la zéolithe oméga ne subit pas désalumination. Elle est soumise dans un premier temps une calcination dite sèche à 550°C sous flux d'air et d'azote durant 6 heures. Puis, le solide obtenu est soumis à trois échanges ioniques dans une solution de $NH_4NO_3$ 10N à environ 100°C pendant 4 heures, pour chaque échange. La forme ammonium de la zéolithe oméga ainsi obtenue est alors calcinée dans un premier temps sous flux d'air sec dilué dans l'azote (0.4l/h/g d'air sec pour 2l/h/g d'azote) durant 2 heures, puis uniquement sous flux d'air sec durant 4 heures à la température de 550°C.

A l'issue de ces traitements, la zéolithe oméga sous forme H ainsi obtenue posséde un rapport Si/Al atomique global égal à 3.25, un rapport Si/Al global de charpente, déterminé par RMN du $^{29}$Si de 3.2, une teneur pondérale en sodium par rapport au poids de zéolithe oméga sèche de 150 ppm, un volume de maille élémentaire de 2,207 nm$^3$, et une capacité d'adsorption en azote mesurée à -196°C et à P/P$_o$=0.19 de 0.22 cm$^3$ liquide/g.

Le mode de greffage de la zéolithe oméga forme H, de rapport Si/Al global égal à 3.2 obtenu précédemment, est réalisé par la voie en phase gazeuse selon les étapes successives suivantes :

- Activation de la zéolithe oméga, par traitement sous gaz inerte à 150°C durant 12 heures, puis diminution de la température à 20°C, sous gaz inerte.
- Calcination des éventuelles traces de composé organiques adsorbés dans la zéolithe sous flux d'air sec (0.4l/h/g) et de gaz inerte (2 l/h/g) durant 2 heures à 550°C, puis uniquement sous flux d'air sec (2 l/h/g) durant 2 heures toujours à 550°C.

La zéolithe est ensuite refroidie sous vide dynamique jusqu'à température ambiante, et mise sous vide statique ($10^{-4}$ Torr). Le tetrabutylétain est alors injecté à 25°C par l'intermédiaire d'un septum.

La température du milieu réactionnel est alors augmentée jusqu'à 150°C, puis maintenue à cette température durant 6 heures. Après greffage, l'excès de $SnBu_4$ présent dans le système est éliminé par purge sous vide dynamique ou sous gaz inerte.

- Le mode de décomposition des fragments organiques (butyles) est identique à celui utilisé pour l'obtention du solide référencé OM2 dans l'exemple 1.

Le solide obtenu à l'issue de ces traitements est référencé OM3 : sa teneur pondérale en étain est de 1.2%, ses autres carctéristiques restent inchangées par rapport à celles de la zéolithe oméga forme H.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A3 ainsi obtenu (dont la teneur pondérale en platine est d'environ 0.12%) sont reportées dans le tableau I.

Le catalyseur A3 après test catalytique est régénéré selon le mode opératoire décrit dans l'exemple 1 et testé de nouveau dans les conditions décrites dans l'exemple 1. Les performances du catalyseur A3R ainsi régénéré sont reportées dans le tableau II.

Exemple 3 : Catalyseur A4 greffé au germanium, conforme à l'invention.

La zéolithe oméga sous forme H de rapport Si/Al global de 11.3, utilisée pour préparer le catalyseur OM4 est la même que celle employée pour la préparation des catalyseurs OM1 et OM2 de l'exemple 1.

On dépose ensuite le germanium sur la surface externe des cristaux de ladite zéolithe oméga forme H suivant la méthode en phase gazeuse. On opère selon les étapes successives suivantes :

- Activation de la zéolithe sous vide dynamique, à la température de 160°C, durant 8 heures. La température est alors augmentée à la vitesse de 2°C/min jusqu'à 450°C, et maintenue durant 9 heures sous vide dynamique. La température est alors abaissée jusqu'à 250°C, température à laquelle le $GeBu_4$ est injecté. La zéolithe oméga est alors laissée sous tension de vapeur de $GeBu_4$ durant 48 heures à 250°C. Après retour à la température ambiante (20°C), les gaz dégagés sont éliminés sous vide dynamique, puis les espèces physisorbées résiduelles sont désorbées sous vide dynamique durant 8 heures à 220°C. Les fragments butyles fixés sur le germanium sont ensuite décomposés par traitement thermique sous atmosphère oxydante. Ce traitement est réalisé en présence d'un mélange d'oxygène et de gaz inerte, à 450°C durant 4 heures.

Le solide obtenu à l'issue de ces traitements est référencé OM4 : sa teneur pondérale en germanium est de 0.31% ses autres caractéristiques restent inchangées par rapport à celles de la zéolithe oméga forme H.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A4 ainsi obtenu (dont la teneur pondérale en platine est d'environ 0.12%) sont reportées dans le tableau I.

Exemple 4 : Catalyseur A5, greffé au zirconium, conforme à l'invention.

La zéolithe oméga, sous forme H, utilisée pour préparer le solide OM5 est la même que celle employée pour pour la préparation les solides OM1 ,OM2 de l'exemple 1 et OM4 de l'exemple 3.

On dépose ensuite le zirconium sur la surface externe des cristaux de ladite zéolithe oméga forme H suivant la méthode en phase gazeuse. On opère selon les étapes successives suivantes :

- Activation de la zéolithe sous vide dynamique de la zéolithe, à la température de 160°C, durant 8 heures. La température est alors augmentée à la vitesse de 2°C/min jusqu'à 450°C, et maintenue durant 14 heures sous vide dynamique.
- Sublimation de $ZrNp_4$ à 70°C sous vide dynamique. - Traitement à 60°C sous vide dynamique durant une heure, afin d'éliminer toute toute trace de $ZrNp_4$.
- Mise sous tension de vapeur d'eau à 25°C (environ 23 mmHg) afin de transformer l'alkyle en groupement hydroxyle. - Traitement à 250°C sous vide dynamique durant 14 heures, afin d'éliminer l'eau résiduelle. Un traitement thermique sous atmosphère oxydante est réalisé en présence d'un mélange d'oxygène et de gaz inerte, à 450°C durant 4 heures.

Le solide obtenu à l'issue de ces traitements est référencé OM5 : sa teneur pondérale en zirconium est de 0.42% ses autres caractéristiques restent inchangées par rapport à celles de la zéolithe oméga forme H.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A5 ainsi obtenu (dont la teneur pondérale en platine est d'environ 0.12%) sont reportées dans le tableau I.

Exemple 5 : Catalyseur A6, non conforme à l'invention.

Le catalyseur A6 renferme la zéolithe oméga forme H de rapport Si/Al global 11.3 utilisée pour la préparation des catalyseurs A1, A2, A4 et A5.

Mais aucun greffage n'est réalisé.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A6 ainsi obtenu (dont la teneur pondérale en platine est d'environ 0.12%) sont reportées dans le tableau I.

Le catalyseur A6 après test catalytique est régénéré selon le mode opératoire décrit dans l'exemple 1 et testé de nouveau dans les conditions décrites dans l'exemple 1. Les performances du catalyseur A6R ainsi régénéré sont reportées dans le tableau II.

Exemple 6 : Catalyseur A7, non conforme à l'invention.

Le catalyseur A7 renferme la zéolithe oméga forme H, de rapport Si/Al global 3.25, utilisée pour la préparation du catalyseur A3. Mais aucun greffage n'est réalisé.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A7 ainsi obtenu (dont la teneur pondérale en platine est d'environ 0.12%) sont reportées dans le tableau I.

Le catalyseur A7 après test catalytique est régénéré selon le mode opératoire décrit dans l'exemple 1 et testé de nouveau dans les conditions décrites dans l'exemple 1. Les performances du catalyseur A7R ainsi régénéré sont reportées dans le tableau II.

Exemple 7 : Catalyseur A8, non conforme à l'invention.

Le catalyseur A8 renferme une mordénite forme H, de rapport Si/Al global 11. Le $SnBu_4$ est greffé sur la surface externe des cristaux de la mordénite forme H, selon la méthode en phase liquide, identique à celle utilisée pour la préparation du catalyseur A1 de l'exemple 1.

De même, le mode de décomposition sous vide des groupements butyles liés à l'étain est le même que celui utilisé lors de la préparation du catalyseur A1 de l'exemple 1. Le solide obtenu à l'issue de ces traitements est référencé OM1 et sa teneur pondérale en étain est de 1.34%.

Les étapes de mélange zéolithe oméga-alumine, de dispersion du platine, de mise en forme, de réduction du catalyseur et les conditions de test d'isomérisation sont identiques à celles décrites dans l'exemple 1.

Les performances du catalyseur A8 ainsi obtenues (dont la teneur pondérale en platine est d'environ 0.12%) sont reportées dans le tableau I. Le catalyseur A8 après test catalytique est régénéré selon le mode opératoire décrit dans l'exemple 1. Les performances du catalyseur A8R sont reportées dans le tableau II.

Effet du greffage sur les sélectivités à iso-approche à l'équilibre

Le tableau I décrit les performances des catalyseurs A1, A2, A3, A4, A5, A6 et A7, comaprés à iso approche à l'équilibre, non régénérés et préparés selon les modes opératoires décrits précédemment. L'effet du greffage sur les sélectivités est particulièrement mis en évidence.

Les catalyseurs A1, A2, A3, A4, A5, A6 et A7 ont été testés dans les conditions décrites précédemment à savoir l'isomérisation d'un mélange orthoxylène (80% poids) et éthylbenzène (20% poids), à une température de 410°C, sous une pression de 12 bars, avec une vitesse spatiale (pph) de 10 (heure)$^{-1}$ et un rapport molaire hydrogène sur hydrocarbures ($H_2$/HC) de 4 environ.

TABLEAU I

| Effet du greffage sur les sélectivités à iso-approche à l'équilibre | | | | | | | | |
|---|---|---|---|---|---|---|---|---|
| Catalyseur | A1 | A2 | A3 | A4 | A5 | A6 | A7 | A8 |
| Exemple | 1 | 1 | 2 | 3 | 4 | 5 | 6 | 7 |
| pph (h$^{-1}$) | 10 | 10 | 10 | 10 | 10 | 10 | 10 | 14 |
| (%) AEQ o-xylène | 90.3 | 91.1 | 90.6 | 91.0 | 90.8 | 90.5 | 91.0 | 91.3 |
| Rendement C$_8$ aromatiques + naphtènes % | 93.8 | 94.1 | 94.3 | 93.7 | 94.6 | 88.2 | 90.5 | 90.2 |
| % Dismutation | 2.9 | 2.8 | 2.1 | 3.0 | 2.1 | 8.3 | 6.1 | 6.5 |
| % Déalkylation | 0.8 | 0.7 | 0.5 | 0.9 | 0.6 | 1.5 | 1.3 | 1.7 |
| % Craquage | 2.1 | 2.2 | 2.0 | 1.7 | 1.4 | 1.9 | 1.8 | 1.6 |

Les catalyseurs A1, A2, A3, A4 et A5 conformes à l'invention sont plus performants que les catalyseurs A6 et A7 de l'art antérieur : le rendement en isomérisation des C$_8$ aromatiques + naphtènes des catalyseurs A1, A2, A3, A4 et A5 est supérieur à celui des catalyseurs A6 et A7. Avec les zéolithes oméga sélectivées selon l'invention (catalyseurs A1, A2, A3, A4 et A5), la réaction secondaire de dismutation conduisant à la formation de triméthylbenzènes est très fortement inhibée par rapport à ce que l'on obtient en présence de zéolithe oméga non sélectivée (catalyseur A6 et A7). D'autre part, on observe qu'une diminution du rapport Si/Al global de la zéolithe conduit aussi à une diminution du taux de dismutation, aussi bien dans le cas de la zéolithe non greffée (catalyseur A7) que dans le cas de la zéolithe greffée (catalyseur A3). Le catalyseur A8 non conforme à l'invention (mordénite greffée) ne pas conduit à un taux de dismutation aussi faible que celui enregistré dans le cas des catalyseur à base de zéolithe oméga greffée. Les résultats obtenus en isomérisation de la coupe C$_8$ aromatique sont donc bien supérieurs à ceux obtenus avec la mordénite greffée et sont tout à fait inattendus.

Des résultats sensiblement les mêmes que ceux enregistrés avec les catalyseurs A1, A2, A3, A4 et A5, sont obtenus en remplaçant l'étain d'abord par du magnésium, puis du titane et enfin du zinc.

Effet des traitement de régénération

Le tableau II décrit les performances des catalyseurs A1R, A6R et A7R contenant la zéolithe oméga et régénérés selon les conditions opératoires décrites dans l'exemple 1, et du catalyseur A8R régénéré et contenant la mordénite. Ces catalyseurs ont été préparés selon les modes opératoires décrits précédemment. L'effet du traitement de régénération sur l'activité catalytique et sur les sélectivités est particulièrement mis en évidence.

Les catalyseurs A1R, A3R, A6R, A7R et A8R ont été testés dans les conditions décrites précédemment à savoir isomérisation d'un mélange orthoxylène (80% poids) et éthylbenzène (20% poids), à une température de 410°C, sous une pression de 12 bars, avec une vitesse spatiale (pph) de 50 (heure)$^{-1}$ et un rapport molaire hydrogène sur hydrocarbures (H$_2$/HC) de 4 environ.

TABLEAU II

| Effet des traitements de régénération à iso-approche à l'équilibre | | | | | |
|---|---|---|---|---|---|
| Catalyseur | A1R | A3R | A6R | A7R | A8R |
| Exemple | 1 | 2 | 5 | 6 | 7 |
| pph (h$^{-1}$) | 50 | 50 | 50 | 50 | 14 |
| AEQ o-xylène | 90.9 | 90.5 | 91.0 | 90.7 | 91.0 |
| Rendement C$_8$ aromatiques + naphtènes % | 94.0 | 94.4 | 88.4 | 90.6 | 90.2 |
| % Dismutation | 2.6 | 1.9 | 8.2 | 6.0 | 6.6 |
| % Déalkylation | 0.7 | 0.9 | 0.4 | 0.8 | 1.8 |
| % Craquage | 2.0 | 1.9 | 2.1 | 1.8 | 1.5 |

Les résultats regroupés dans le tableau II montrent, que d'une façon surprenante, les traitements de régénération effectués sur les catalyseurs à base de zéolithes oméga greffées à l'étain (A1R et A3R) sont beaucoup plus actifs que les catalyseurs non régénérés (A1 et A3). En effet, dans le cas des catalyseurs à base de zéolithe oméga et non régénérés une pph de 10 $h^{-1}$ conduit à une approche à l'équilibre en ortho-xylène de l'ordre de 90%, une telle valeur, est obtenue en imposant une pph de 50 $h^{-1}$, dans le cas des catalyseurs A1R et A3R régénérés. L'activité se trouve donc multipliée par un facteur 5. Par ailleurs, on observe aussi de façon surprenante que les taux de dismutation enregistrés dans le cas des catalyseurs à base de zéolithes oméga greffées à l'étain (A1R et A3R) sont plus faibles que ceux obtenus dans le cas des catalyseurs non régénérés. De tels effets, dus aux traitements de régénération, ne sont pas observés dans le cas des catalyseurs à base de zéolithe oméga non greffée et dans le cas du catalyseur A8R à base de mordénite greffée via $SnBu_4$. En effet, pour le catalyseur A8R régénéré on observe qu'il est nécessaire de maintenir une pph de 14 ($h^-$) pour obtenir des preformances catalytiques comparables à celles obtenues avec le catalyseur A8 non régénéré et, testé lui aussi à pph 14.

Des effets analogues sur l'activité catalytique et le taux de dismutation, après traitements de régénération, ont été observés dans le cas des catalyseurs à base de zéolithe oméga greffées au germanium et au zirconium.

## Revendications

1. Catalyseur renfermant une matrice, au moins un métal du GVIII et une zéolithe oméga contenant au moins un métal choisi parmi les métaux des groupes IIa, IIb, IVa et IVb et telle que :

   - son rapport atomique Si/Al global est compris entre 2.5 et 100,
   - sa teneur pondérale en sodium par rapport au poids de zéolithe oméga sèche est inférieure à 2000 ppm, caractérisé en ce que:

     le (ou les) métal(aux) sont déposés sur la zéolithe oméga par greffage avec au moins un composé organométallique dudit (desdits) métal(aux).

2. Catalyseur selon la revendication 1, caractérisé en qu'il est obtenu par le procédé consistant à déposer par greffage, sur la zéolithe oméga, au moins un des métaux des groupes IIa, IVb, IIb et IVa puis, à mélanger la zéolithe oméga greffée avec une matrice contenant un métal du groupe VIII.

3. Catalyseur selon l'une des revendications 1 à 2, caractérisé en ce qu'il est obtenu par le procédé consistant à déposer par greffage sur la zéolithe oméga, au moins un métal choisi parmi les métaux des groupes IIa, IVb, IIb et IVa, puis à mélanger la zéolithe greffée avec une matrice et à réaliser ensuite le dépôt du métal du groupe VIII

4. Catalyseur selon l'une des revendications 1 à 3, caractérisé en ce qu'il est obtenu par le procédé consistant à déposer par greffage sur la zéolithe oméga, au moins un métal choisi parmi les métaux des groupes IIa, IVb, IIb et IVa, ledit dépôt étant précédé ou suivi du dépôt d'au moins un métal du groupe VIII, la zéolithe greffée et chargée en métal du groupe VIII étant ensuite mélangée à la matrice.

5. Catalyseur selon l'une des revendications 1 à 4, caractérisé en ce que le greffage a lieu en phase liquide, le composé organométallique étant en solution dans un solvant, ledit solvant étant un alcane possédant au moins 5 atomes de carbone.

6. Catalyseur selon l'une des revendications 1 à 5, caractérisé en ce que le métal greffé est choisi dans le groupe formé par l'étain, le magnésium, le germanium et le zirconium.

7. Catalyseur selon l'une des revendications 1 à 6, caractérisé en ce que la teneur pondérale en métal des groupes IIa, IIb, IVa, IVb de ladite zéolithe oméga est comprise entre 0,01 et 25%.

8. Catalyseur selon l'une des revendications 1 à 7, caractérisé en ce que le métal du GVIII est choisi dans le groupe formé par le palladium et le platine.

9. Catalyseur selon l'une des revendications 1 à 8, caractérisé en ce que la matrice est choisi dans le groupe formé par l'alumine, la magnésie, la silice-alumine, les argiles naturelles.

10. Procédé d'isomérisation d'une coupe $C_8$ aromatique à une température comprise entre 270°C et 600°C, une pression de 0.5 à 100 bars, une vitesse spatiale de charge par unité de charge de catalyseur et par heure comprise entre 0.5 et 200 et un rapport molaire $H_2$/hydrocarbure compris entre 0.5 et 12, procédé dans lequel est utilisé un catalyseur à base de zéolithe oméga, obtenu selon l'une des revendications 1 à 9.

11. Procédé selon la revendication 12, dans lequel le catalyseur est régénéré après avoir été mis en contact avec la charge dans les conditions d'isomérisation de la coupe $C_8$ aromatique.

**Claims**

1. A catalyst containing a matrix, at least one group VIII metal and an omega zeolite containing at least one metal selected from group IIa, IIb, IVa and IVb metals and such that:

   - its global Si/Al atomic ratio is between 2.5 and 100,
   - its content by weight of sodium in relation to the weight of dry omega zeolite is less than 2000 ppm, characterised in that:

     the metal(s) is/are deposited on the omega zeolite by grafting with at least one organometallic compound of said metal(s).

2. A catalyst according to Claim 1, characterised in that it is obtained by the process consisting of depositing by grafting on the omega zeolite at least one of the group IIa, IVb, IIb and IVa metals, and then in mixing the grafted omega zeolite with a matrix containing a group VIII metal.

3. A catalyst according to one of Claims 1 to 2, characterised in that it is obtained by the process consisting of depositing by grafting on the omega zeolite at least one metal selected from group IIa, IVb, IIb and IVa metals, and then in mixing the grafted zeolite with a matrix and in then depositing the group VIII metal.

4. A catalyst according to one of Claims 1 to 3, characterised in that it is obtained by the process consisting of depositing by grafting on the omega zeolite at least one metal selected from group IIa, IVb, IIb and IVa metals, said depositing being preceded by, or followed by, the depositing of at least one group VIII metal, the grafted zeolite which is charged with the group VIII metal then being mixed with the matrix.

5. A catalyst according to one of Claims 1 to 4, characterised in that the grafting operation takes place in liquid phase, the organometallic compound being in solution in a solvent, said solvent being an alkane with at least 5 carbon atoms.

6. A catalyst according to one of Claims 1 to 5, characterised in that the grafted metal is selected from the group formed by tin, magnesium, germanium and zirconium.

7. A catalyst according to one of Claims 1 to 6, characterised in that the content by weight of group IIa, IIb, IVa, IVb metals of said omega zeolite is between 0.01 and 25%.

8. A catalyst according to one of Claims 1 to 7, characterised in that the group VIII metal is selected from the group formed by palladium and platinum.

9. A catalyst according to one of Claims 1 to 8, characterised in that the matrix is selected from the group formed by alumina, magnesium oxide, silica-alumina, and natural clays.

10. A process for isomerisation of a $C_8$ aromatic cut at a temperature of between 270°C and 600°C, a pressure of 0.5 to 100 bars, a spatial speed of charge per unit of charge of catalyst and per hour of between 0.5 and 200 and a $H_2$/hydrocarbon molar ratio of between 0.5 and 12, in which process a catalyst is used which has an omega zeolite base and which is obtained according to one of Claims 1 to 9.

11. A process according to Claim 10, wherein the catalyst is regenerated after having been placed in contact with the charge under isomerisation conditions of the aromatic $C_8$ cut.

**Patentansprüche**

1. Katalysator, enthaltend eine Matrix, wenigstens ein Metall der Gruppe VIII und einen Omega-Zeolith, der wenigstens ein Metall enthält, das unter den Metallen der Gruppen IIa, IIb, IVa und IVb ausgewählt ist und derart ist, daß:

- dessen gesamtes Atomverhältnis Si/Al einschließlich zwischen 2,5 und 100 beträgt,
- dessen gewichtsanalytischer Gehalt an Natrium in bezug auf das Gewicht von trockenem Omega-Zeolith kleiner als 2000 ppm ist,
dadurch gekennzeichnet, daß:

das (oder die) Metall(e) auf dem Omega-Zeolith durch Veredelung mit wenigstens einem metallorganischen Bestandteil des Metalls (der Metalle) niedergeschlagen ist (sind).

2. Katalysator nach Anspruch 1, dadurch gekennzeichnet, daß er durch das Verfahren erhalten ist, welches darin besteht, durch Veredelung auf dem Omega-Zeolith wenigstens eines der Metalle der Gruppen IIa, IVb, IIb und IVa niederzuschlagen, dann den veredelten Omega-Zeolith mit einer Matrix zu mischen, die ein Metall der Gruppe VIII enthält.

3. Katalysator nach einem der Ansprüche 1 bis 2, dadurch gekennzeichnet, daß er durch das Verfahren erhalten ist, welches darin besteht, durch Veredelung auf dem Omega-Zeolith wenigstens ein Metall, das unter den Metallen der Gruppen IIa, IVb, IIb und IVa ausgewählt ist, niederzuschlagen, dann den veredelten Zeolith mit einer Matrix zu mischen und danach den Niederschlag des Metalls der Gruppe VIII zu verwirklichen.

4. Katalysator nach einem der Ansprüche 1 bis 3, dadurch gekennzeichnet, daß er durch das Verfahren erhalten ist, welches darin besteht, durch Veredelung auf dem Omega-Zeolith wenigstens ein Metall, das unter den Metallen der Gruppen IIa, IVb, IIb und IVa ausgewählt ist, niederzuschlagen, wobei der Niederschlag dem Niederschlag von wenigstens einem Metall der Gruppe VIII vorausgeht oder nachfolgt, der veredelte und mit einem Metall der Gruppe VIII beladene Zeolith dann mit der Matrix gemischt wird.

5. Katalysator nach einem der Ansprüche 1 bis 4, dadurch gekennzeichnet, daß die Veredelung in einer Flüssigphase stattfindet, wobei der metallorganische Bestandteil in einem Lösungsmittel in Lösung ist, wobei das Lösungsmittel ein Alkan ist, das wenigstens 5 Kohlenstoffatome besitzt.

6. Katalysator nach einem der Ansprüche 1 bis 5, dadurch gekennzeichnet, daß das veredelte Metall aus der Gruppe ausgewählt ist, die durch das Zinn, das Magnesium, das Germanium und das Zirkonium gebildet ist.

7. Katalysator nach einem der Ansprüche 1 bis 6, dadurch gekennzeichnet, daß der gewichtsanalytische Gehalt an Metall der Gruppen IIa, IIb, IVa, IVb des Omega-Zeoliths einschließlich zwischen 0,01 und 25 % beträgt.

8. Katalysator nach einem der Ansprüche 1 bis 7, dadurch gekennzeichnet, daß das Metall der Gruppe VIII aus der Gruppe ausgewählt ist, die durch das Palladium und das Platin gebildet ist.

9. Katalysator nach einem der Ansprüche 1 bis 8, dadurch gekennzeichnet, daß die Matrix aus der Gruppe ausgewählt ist, die durch das Aluminiumoxid, das Magnesium, das Siliziumdioxid-Aluminiumoxid, die natürlichen Tone gebildet ist.

10. Verfahren zur Isomerisierung eines aromatischen $C_8$-Schnittes bei einer Temperatur einschließlich zwischen 270°C und 600°C, einem Druck von 0,5 bis 100 bar, einer Chargenraumgeschwindigkeit pro Katalysatorchargeneinheit und pro Stunde einschließlich zwischen 0,5 und 200 sowie einem Molverhältnis $H_2$/Kohlenwasserstoff einschließlich zwischen 0,5 und 12, wobei in diesem Verfahren ein Katalysator auf Basis eines Omega-Zeoliths, der nach einem der Ansprüche 1 bis 9 erhalten wird, verwendet wird.

11. Verfahren nach Anspruch 10, bei welchem der Katalysator nach Inkontaktbringung mit der Charge unter den Bedingungen zur Isomerisierung des aromatischen $C_8$-Schnittes regeneriert wird.